(19) Europäisches Patentamt / European Patent Office / Office européen des brevets

(11) **EP 4 710 844 A1**

(12) **EUROPEAN PATENT APPLICATION**

(43) Date of publication:
**18.03.2026 Bulletin 2026/12**

(21) Application number: 24425045.2

(22) Date of filing: **17.09.2024**

(51) International Patent Classification (IPC):
**A61B 5/00** (2006.01)     **G06N 3/0464** (2023.01)

(52) Cooperative Patent Classification (CPC):
**G06N 3/0464; A61B 5/6802; G06N 3/045**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Luxottica S.r.l.**
**32021 Agordo (BL) (IT)**

(72) Inventors:
• **Trojaniello, Diana**
  **32021 Agordo (BL) (IT)**
• **Ongarello, Tommaso**
  **32021 Agordo (BL) (IT)**

• **Shalby, Hazem Hesham Yousef**
  **20133 Milano (MI) (IT)**
• **De Vecchi, Arianna**
  **20133 Milano (MI) (IT)**
• **Villa, Federica**
  **20133 Milano (MI) (IT)**
• **Roveri, Manuel**
  **20133 Milano (MI) (IT)**
• **Palermo, Francesca**
  **32021 Agordo (BL) (IT)**

(74) Representative: **Biallo, Dario et al**
**Barzanò & Zanardo S.p.A.**
**Via Borgonuovo, 10**
**20121 Milano (IT)**

(54) **SMART SENSOR, WEARABLE DEVICE COMPRISING SAID SMART SENSOR AND METHOD FOR PROCESSING THE DATA DETECTED THROUGH SAID SMART SENSOR**

(57)     Smart sensor (200) comprising:
- a detector (210) configured for continuously detecting a physical quantity, sampled with a predefined sampling time $t_c$, thus obtaining a plurality of the detected physical quantity samples $x_{t-i}$ with $i \in [0, T-1]$ of size $N_{in}$ where
• $x_{t-i} \in R^{Nin}$ with $i \in [0, T-1]$ is the sample acquired at discrete time $t-i$ and is a vector of size $N_{in}$,
• T is the number of samples acquired in a predetermine time size $T \times t_c$ of an observation window;

- an embedded first electronic unit (220) configured for receiving the acquired samples $x_{t-i}$ with $i \in [0, T-1]$ and for executing, each time a new sample $x_{t-i}$ with $i \in [0, T-1]$ is received, an incremental Neural Network $g^{(incr)}(\cdot)$ having such a received new sample $x_{t-i}$ with $i \in [0, T-1]$ as an input, wherein such an incremental Neural Network $g^{(incr)}(\cdot)$ provides the application of a number of n convolutional filters to each sample $x_{t-i}$ obtaining a corresponding first output output matrix Y having elements $y_{t-i}^{(j,k)}$ with $j \in [1, n]$ and $k \in [1, N_{in}]$ according to the following equation:

$$y_{t-i}^{(j,k)} = y_{t-i-1}^{(j,k)} + c_i^j \times x_{t-i}$$

$\forall j \in [1, n]$ and $\forall k \in [1, N_{in}]$ wherein $c_i^j$ is the weight of the j-th filter applied to the sample at time t-i, said first output matrix Y having size n x $N_{in}$ and being initialized to 0, and wherein the embedded first electronic unit (220) is configured to transmit the first output matrix Y to a second electronic unit (300) if a predefined condition is satisfied.

Fig. 1

**Description**

The present invention refers to a smart sensor, to a wearable device comprising said smart sensor and to a method for processing data detected from said smart sensor.

Smart sensors are currently known which comprises a detector, for example the detector is an Inertial Measurement Unit (IMU), that is capable of detecting a physical quantity, like an acceleration, a temperature, a pressure and so on, and an embedded first electronic unit capable of receiving, processing and/or storing the data detected through the detectors.

In particular, the detected data are a plurality of samples of the physical quantity detected. In particular, the first electronic unit is configured for storing a predefined number of the samples detected in a time window of a predefined duration. The first electronic unit is usually a microprocessor with low computational capacity in order to maintain the cost of the smart sensor compatible with the market. The first electronic unit, then, may be configured for performing some simple calculation on the stored samples, like the calculation of the arithmetical mean and variance.

In any case, the known smart sensors are configured for sending the stored samples to an external second electronic unit that is usually configured for performing complex calculations with higher computational cost based on the received samples in order to extract useful informations.

In particular, the second electronic unit may be programmed to implement an artificial intelligence algorithm like for example a Convolutional Neural Network (CNN) capable of determining some parameters starting from the received detected samples. These kind of algorithms is capable of very accurate calculations that may be required in some application scenarios for example for the human activity recognition. Specifically, a CNN can be formulated as a function $f(x_t, \ldots, x_{t-(T-1)})$ where:

- $xt \in R^{Nin}$ is the sample acquired at time t of size $N_{in}$,
- T is the the number of samples acquired in the window on which $f(\cdot)$ is performed.

This algorithm is performed on a plurality of samples and, thus, may require high computational resources and time. In this case, the second electronic unit needs to be active for long time intervals due to the high data quantity that are transmitted by the first electronic unit, thus resulting in a high energy consumption of the second electronic unit.

In case such a CNN network is implemented in the first electronic unit, if the calculation takes more time than the time between two samples , it is possible that some new samples get lost since they cannot be processed by the busy first electronic unit. Therefore the use of a CNN network, if performed on the first electronic unit, may be accompanied by some strict requirements in terms of calculation time that are very difficult to be satisfied.

It is currently known to provide a smart sensor like those above described in a wearable device, for example for human activity recognition or for detecting if such a wearable device is worn by a user and for consequently triggering another function of the wearable device depending on the previous detection. For example, a wearable device may be an eyewear, a generic head-mountable device, a smartwatch and so on. Head-mountable devices encompasses any type of device designed to be worn over at least one eye of a wearer and having a frame configured to be mounted on a head, including any eyewear.

In these cases the wearable devices usually comprise also a microcontroller that may act as the above described second electronic unit in addition to performing some other functions that nowadays are more and more required to the wearable devices, such as the localization and the running of software applications of common use.

In this application scenario it is essential to maintain low the power consumption of such a microcontroller that is however a requirement in conflict with the high computational capability above cited.

The object of the present invention is to overcome the above-mentioned drawbacks and in particular to devise a smart sensor capable of implementing part of a CNN network in the processing unit included of the smart sensor assuring a low energy consumption of the second electronic unit.

Another object of the present invention is to provide a method for processing the data detected through said smart sensor that allows the reduction of the memory occupation and of the inference time of the processing unit of the smart sensor avoiding the sample loss.

This and other objects according to the present invention are achieved by making a smart sensor according to claim 1, a wearable device according to claim 4 and a method for processing data detected from said smart sensor according to claim 8.

Further characteristics of the smart sensor, of the wearable device and of the method for processing data detected from said smart sensor are the objects of the dependent claims.

The characteristics and advantages of the smart sensor, of the wearable device and of the method for processing data detected from said smart sensor according to the present invention will be more evident from the following exemplary though non-limiting description, referring to the attached schematic drawings in which:

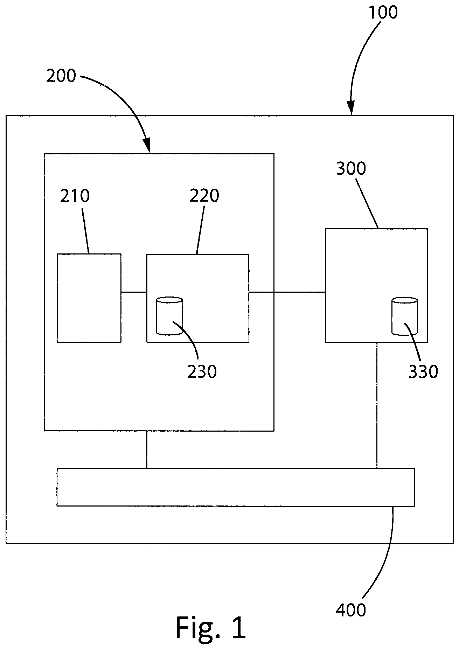

- Figures 1 is a block scheme of an embodiment of a wearable device according to the present invention;

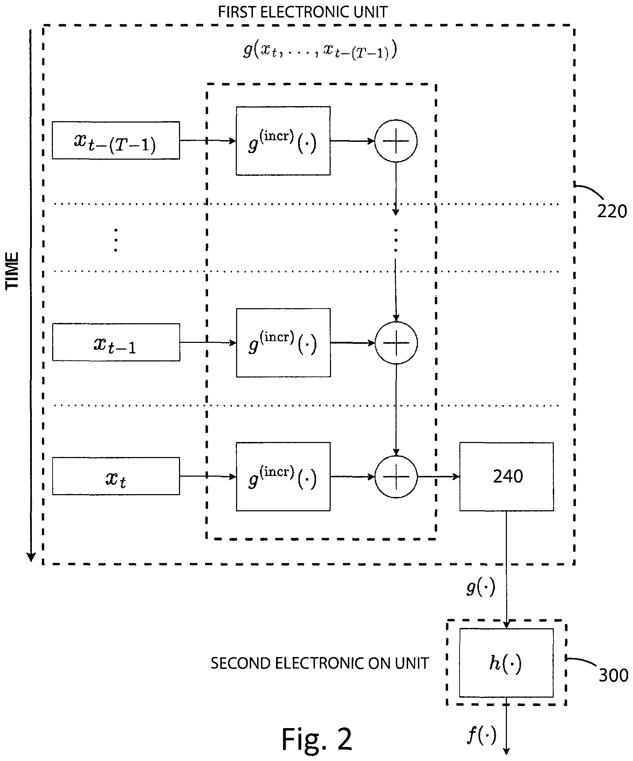

- Figure 2 is a representation of the operations performed by a first electronic unit and a second electronic unit according to the present invention. With reference to the figures, a wearable device is shown, globally referred to as 100.

For example, the wearable device 100 may be an eyewear, a generic head-mountable device, a smartwatch and so on.

Head-mountable devices encompasses any type of device designed to be worn over at least one eye of a wearer and having a frame configured to be mounted on a head, including any eyewear.

For example, the head-mountable device may be a helmet, or a visor, or a mask or an eyewear and so on.

In particular, in the illustrated figures the wearable device 100 is an eyewear.

The wearable device 100 comprises a supporting structure 110 adapted to be worn by a user. Advantageously, the wearable device 100 comprises a smart sensor 200 according to the present invention. The smart sensor 200 comprises a detector 210 configured for continuously detecting a physical quantity, sampled with a predefined sampling time $t_c$, thus obtaining a plurality of the detected physical quantity samples $x_{t-i}$ with $i \in [0, T-1]$ of size $N_{in}$ where

- $x_{t-i} \in R^{N_{in}}$ with $i \in [0, T-1]$ is the sample acquired at discrete time t-i and is a vector of size $N_{in}$,
- T is the number of samples acquired in a predetermined time size $T \times t_c$ of an observation window.

For example $t_c$ may be equal to 10 ms, whereas T may be equal to 1000, or 500, or 200 or 100, thus resulting in a predetermined time size of the observation window $T \times t_c$ equal to 10 s, or 5 s, or 2 s, or 1 s.

Preferably, the detector 210 may be a monoaxial accelerometer capable of detecting accelerations along one axis of the tridimensional Cartesian reference system, or a triaxial accelerometer capable of detecting accelerations along three axis of the tridimensional Cartesian reference system, or a gyroscope.

More preferably, the detector 210 may be an Inertial Measurement Unit (IMU).

Preferably, the detector 210 may be a temperature sensor.

More generally the detector 210 may be capable of detecting a motion quantity or an environmental condition.

In any case the detector is of digital type capable of outputting samples of the detected physical quantity. The smart sensor 200 comprises also an embedded first electronic unit 220 configured for receiving the acquired samples $x_{t-i}$ with $i \in [0, T-1]$ and for executing, each time a new sample $x_{t-i}$ with $i \in [0, T-1]$ is received, an incremental first Neural Network $g^{(incr)}(\cdot)$ having such a received new sample $x_{t-i}$ with $i \in [0, T-1]$ as an input, wherein such an incremental first Neural Network $g^{(incr)}(\cdot)$ provides the application of a number of n convolutional filters to each sample $x_{t-i}$ obtaining a corresponding first output matrix Y having elements $y_{t-i}^{(j,k)}$ with $j \in [1, n]$ and $k \in [1, N_{in}]$ according to the following equation:

$$y_{t-i}^{(j,k)} = y_{t-i-1}^{(j,k)} + c_i^j \times x_{t-i}$$

$\forall j \in [1, n]$ and $\forall k \in [1, N_{in}]$

wherein $c_i^j$ is the weight of the j-th filter applied to the sample at time i.

Such a first output matrix Y has size $n \times N_{in}$ and is initialized to 0.

Preferably, each convolutional filter is a one-dimensional convolutional filter with a kernel size of $1 \times 1 \times T$.

In this description, with the term "electronic unit" it is intended to indicate at least a processing unit, like a microprocessor, or even a processing and control unit like a microcontroller.

Moreover, the smart sensor 200 is configured to communicate with a second electronic unit 300.

In particular, the second electronic unit may be connected to the first electronic unit 220 through one or more cables.

In any case, the first electronic unit 220 is configured to transmit the first output matrix Y to the second electronic unit 300 if a predefined condition is satisfied.

In particular, the first electronic unit is provided with a early exit module 240 configured for verifying that the predefined condition is satisfied; such an early exit module 240 is executed by the first electronic unit 220. The early exit module 240 may be, for example, a firmware module.

For example the early exit module 240 is configured to compare the elements $y_{t-i}^{(j,k)}$ with respective thresholds in order to verify the predefined conditions (greater than, or less than). In such a case, for example, the thresholds may be chosen so as to be capable of distinguishing whether the user is wearing or not the wearable device.

Alternatively, the early exit module 240 may be implemented as a neural network classifier module capable of extracting from the output matrix Y the indication of a predefined condition. In this latter case, for example, the predefined condition may be indicative of whether the user is wearing or not the wearable device.

This could be useful since it is possible to trigger the actuation of some software applications through the detection of whether the user is wearing or not the wearable device.

For example, the detector is an IMU with $N_{in}=6$ and the incremental first Neural Network $g^{(incr)}(\cdot)$ is implemented through two 1D convolutions with n = 16, thus obtaining an output matrix Y having dimensions 16x6; in this case, for example, the early exit module 240 is implemented as a Max Pooling layer of size 6 followed by a fully connected layer of dimension 2 for classifying the output of the early exit module as worn/not worn device. The second electronic unit 300 may be external to the wearable device 100, for example comprised in a personal computer, or in a laptop, or in mobile terminal like a smartphone or a smartwatch. Alternatively, the second electronic unit 300 may be integrated in the wearable device 100.

Moreover, the second electronic unit 300 is configured for executing a second Neural Network $h(\cdot)$ obtaining a second output matrix representing a condition of the user wearing the wearable device.

The first electronic unit 220 and the second electronic unit 300 may comprise a respective internal memory 230, 330 that may be used also for locally storing the data received or processed.

Preferably, the condition of the user is indicative of an activity that the user is doing. In this case the smart sensor 200 and the second electronic unit 300 realizes a human activity recognition system. Preferably the wearable device 100 comprise also a battery 400 suitable for electrically supply the smart sensor 200 and the second electronic unit 300 if integrated in the wearable device 100.

The second Neural Network $h(\cdot)$ can be any general task performed on the output of the incremental first Neural Network $g^{(incr)}(\cdot)$.

In any case, the sequence of the first Neural Network $g^{(incr)}(\cdot)$ and of the second Neural Network $h(\cdot)$ constitutes the CNN $f(\cdot)=h(g^{(incr)}(\cdot))$.

The training of the CNN $f(\cdot)$ is done as follows:

- $f(.)$ is optimized in an end-to-end manner for a given task, determining the weights of both the first Neural Network $g^{(incr)}(\cdot)$ and the second Neural Network $h(\cdot)$;
- the weights of $g^{(incr)}(\cdot)$ are then fixed and used to train the early exit module.

In the following it will be described a method for processing data according to the present invention that can be implemented by the smart sensor and the second electronic unit above cited.

Such a method for processing data comprises the steps:

- receiving the data related to a physical quantity detected and sampled with a predefined sampling time $t_c$ through a detector, wherein said data are a plurality of the detected physical quantity samples $x_{t-i}$ with $i \in [0, T-1]$ of size $N_{in}$ where

  • $x_{t-i} \in R^{N_{in}}$ with $i \in [0, T-1]$ is the sample acquired at discrete time $t-i$ and is a vector of size $N_{in}$,
  • $T$ is the number of samples acquired in a predetermine time size $T \times t_c$ of an observation window;

- executing through a first electronic unit, each time a new sample $x_{t-i}$ with $i \in [0, T-1]$ is received, an incremental first Neural Network $g^{(incr)}(\cdot)$ having such a received new sample $x_{t-i}$ with $i \in [0, T-1]$ as an input, wherein such an incremental first Neural Network $g^{(incr)}(\cdot)$ provides the application of a number of n convolutional filters to each sample $x_{t-i}$ obtaining a corresponding first output output matrix Y having elements $y_{t-i}^{(j,k)}$ with $j \in [1, n]$ and $k \in [1, N_{in}]$ according to the following equation:

$$y_{t-i}^{(j,k)} = y_{t-i-1}^{(j,k)} + c_i^j \times x_{t-i}$$

$\forall j \in [1, n]$ and $\forall k \in [1, N_{in}]$

wherein $c_i^j$ is the weight of the j-th filter applied to the sample at time I, said first output matrix Y having size $n \times N_{in}$ being initialized to 0;
- transmitting through the first electronic unit the first output matrix Y to a second electronic unit if a predefined condition is satisfied.

Preferably the method for processing data comprises the step:

- executing through the second electronic unit a second Neural Network $h(\cdot)$ obtaining a second output matrix. From the description made, the characteristics of the smart sensor, to the wearable device and to the method for processing data, that are object of the present invention, are clear, as are the relative advantages. The smart sensor is capable of implementing the first Neural Network that is part of the CNN network in a quicker way with respect to the prior art. Indeed the first Neural Network is executed according to an incremental approach on each one of the detected samples once they are received by the first electronic unit differently from the prior art wherein the CNN was applied on all the detected samples.

The execution of the incremental first Neural Network results to be lighter from a computational point of view and therefore it can be performed by the first electronic unit that is an embedded electronic unit with limited hardware resources.

Moreover, since the execution of the incremental first Neural Network is performed each time a new sample is received and in view of its light computational cost the risk to lose samples is reduced and both the memory consumption and inference time results to be independent of the input size and so of the observation time interval.

The second electronic unit is actuated only when the predefined condition satisfaction is verified by the early exit module assuring a reduced power consumption of the same second electronic unit.

Finally, it is clear that the smart sensor, the wearable device and the method for processing data thus conceived is susceptible of numerous modifications and variations, all of which are within the scope of the invention; moreover, all the details may be replaced by technically equivalent elements. In practice, the materials used, as well as their dimensions, can be of any type according to the technical requirements.

## Claims

1. Smart sensor (200) comprising:

   - a detector (210) configured for continuously detecting a physical quantity, sampled with a predefined sampling time $t_c$, thus obtaining a plurality of the detected physical quantity samples $x_{t-i}$ with $i \in [0, T-1]$ of size $N_{in}$ where

     • $x_{t-i} \in R^{Nin}$ with $i \in [0, T-1]$ is the sample acquired at discrete time t-i and is a vector of size $N_{in}$,
     • T is the number of samples acquired in a predetermine time size T x $t_c$ of an observation window;

   - an embedded first electronic unit (220) configured for receiving the acquired samples $x_{t-i}$ with $i \in [0, T-1]$ and for executing, each time a new sample $x_{t-i}$ with i6[0, T-1] is received, an incremental Neural Network $g^{(incr)}(\cdot)$ having such a received new sample $x_{t-i}$ with i6[0, T-1] as an input, wherein such an incremental Neural Network $g^{(incr)}(\cdot)$ provides the application of a number of n convolutional filters to each sample $x_{t-i}$ obtaining a corresponding first output matrix Y having elements $y_{t-i}^{(j,k)}$ with $j \in [1, n]$ and $k \in [1, N_{in}]$ according to the following equation:

$$y_{t-i}^{(j,k)} = y_{t-i-1}^{(j,k)} + c_i^j \times x_{t-i}$$

   $\forall j \in [1, n]$ and $\forall k \in [1, N_{in}]$

   wherein $c_i^j$ is the weight of the j-th filter applied to the sample at time t-i, said first output matrix Y having size n x $N_{in}$ and being initialized to 0, and wherein the embedded first electronic unit (220) is configured to transmit the first output matrix Y to a second electronic unit (300) if a predefined condition is satisfied.

2. Smart sensor (200) according to claim 1 wherein the detector (210) is an Inertial Measurement Unit.

3. Smart sensor (200) according to one or more of the previous claims wherein the first electronic unit (220) is provided with a early exit module (240) configured for verifying that the predefined condition is satisfied, said early exit module (240) being executed by the first electronic unit (220).

4. Wearable device (100) comprising:

   - a smart sensor (200) in turn comprising

     - a detector (210) configured for continuously detecting a physical quantity, sampled with a predefined sampling time $t_c$, thus obtaining a plurality of detected physical quantity samples $x_{t-i}$ with $i \in [0, T-1]$ of size $N_{in}$ where

       • $x_{t-i} \in R^{Nin}$ with $i \in [0, T-1]$ is the sample acquired at discrete time t-i and is a vector of size $N_{in}$,
       • T is the number of samples acquired in a predetermine time size T x $t_c$ of an observation window;

     - an embedded first electronic unit (220) configured for receiving the acquired samples $x_{t-i}$ with $i \in [0, T-1]$ and for executing, each time a new sample $x_{t-i}$ with $i \in [0, T-1]$ is received, an incremental first Neural Network $g^{(incr)}(\cdot)$ having such a received new sample $x_{t-i}$ with $i \in [0, T-1]$ as an input, wherein such an incremental first Neural Network $g^{(incr)}(\cdot)$ provides the application of a number of n convolutional filters to each sample $x_{t-i}$ obtaining a corresponding first output output matrix Y having elements $y_{t-i}^{(j,k)}$ with $j \in [1, n]$ and $k \in [1, N_{in}]$ according to the following equation:

$$y_{t-i}^{(j,k)} = y_{t-i-1}^{(j,k)} + c_i^j \times x_{t-i}$$

     $\forall j \in [1, n]$ and $\forall k \in [1, N_{in}]$

     wherein $c_i^j$ is the weight of the j-th filter applied to the sample at time t-i, said first output matrix Y having size n x Nin and being initialized to 0; wherein the first electronic unit (220) is configured to transmit the first output matrix Y to a second electronic unit (300) if a predefined condition is satisfied.

**5.** Wearable device (400) according to claim 4 wherein the second electronic unit (300) is integrated in the wearable device (100) and is configured for executing a second Neural Network h(·) obtaining a second output matrix representing a condition of the user wearing the wearable device.

**6.** Wearable device according to claim 5 wherein the condition of the user is indicative of an activity that the user is doing.

**7.** Wearable device according to one or more of claims from 4 to 6 wherein the predefined condition is indicative of whether the user is wearing or not the wearable device.

**8.** Method for processing data comprising the steps:

- receiving the data related to a physical quantity detected and sampled with a predefined sampling time $t_c$ through a detector (210), wherein said data are a plurality of the detected physical quantity samples $x_{t-i}$ with i6[0, T-1] of size $N_{in}$ where

• $x_{t-i}$ E $R^{Nin}$ with i∈[0, T-1] is the sample acquired at discrete time t-i and is a vector of size $N_{in}$,
• T is the number of samples acquired in a predetermine time size T × $t_c$ of an observation window;

- executing through a first electronic unit (220), each time a new sample $x_{t-i}$ with i∈[0, T-1] is received, an incremental first Neural Network $g^{(incr)}(·)$ having such a received new sample $x_{t-i}$ with i∈[0, T-1] as an input, wherein such an incremental first Neural Network $g^{(incr)}(·)$ provides the application of a number of n convolutional filters to each sample $x_{t-i}$ obtaining a corresponding first output output matrix Y having elements $y_{t-i}^{(j,k)}$ with j∈[1, n] and k E [1, $N_{in}$] according to the following equation:

$$y_{t-i}^{(j,k)} = y_{t-i-1}^{(j,k)} + c_i^j \times x_{t-i}$$

∀j E [1, n] and ∀k ∈ [1, $N_{in}$]

wherein $c_i^j$ is the weight of the j-th filter applied to the sample at time t-i, said first output matrix Y having size n x $N_{in}$ and being initialized to 0;
- transmitting through the first electronic unit (220) the first output matrix Y to a second electronic unit (300) if a predefined condition is satisfied.

**9.** Method for processing data according to claim 8 further comprising the step:

- executing through the second electronic unit (300) a second Neural Network h(·) obtaining a second output matrix.

Fig. 1

FIRST ELECTRONIC UNIT

$$g\left(x_t, \ldots, x_{t-(T-1)}\right)$$

$$x_{t-(T-1)}$$

$$g^{(\mathrm{incr})}(\cdot)$$

220

$$x_{t-1}$$

$$g^{(\mathrm{incr})}(\cdot)$$

$$x_t$$

$$g^{(\mathrm{incr})}(\cdot)$$

240

TIME

$$g(\cdot)$$

SECOND ELECTRONIC ON UNIT

$$h(\cdot)$$

300

$$f(\cdot)$$

## Fig. 2

**EUROPEAN SEARCH REPORT**

Application Number

EP 24 42 5045

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/104039 A1 (ROBERTSON JOHN JAMES [US] ET AL) 2 April 2020 (2020-04-02) * paragraphs [0001] - [0124] * | 1-9 | INV.<br>A61B5/00<br>G06N3/0464 |
| A | NOVAC PIERRE-EMMANUEL ET AL: "UCA-EHAR: A Dataset for Human Activity Recognition with Embedded AI on Smart Glasses", APPLIED SCIENCES, vol. 12, no. 8, 11 April 2022 (2022-04-11), page 3849, XP093246399, ISSN: 2076-3417, DOI: 10.3390/app12083849 * pages 1-22 * | 1-9 | |
| A | YI MYUNG-KYU ET AL: "A Human Activity Recognition Method Based on Lightweight Feature Extraction Combined With Pruned and Quantized CNN for Wearable Device", IEEE TRANSACTIONS ON CONSUMER ELECTRONICS, IEEE SERVICE CENTER, NEW YORK, NY, US, vol. 69, no. 3, 11 April 2023 (2023-04-11), pages 657-670, XP011946754, ISSN: 0098-3063, DOI: 10.1109/TCE.2023.3266506 [retrieved on 2023-04-12] * pages 657-668 * | 1-9 | |
| A | US 2023/134166 A1 (KALE POORNA [US]) 4 May 2023 (2023-05-04) * paragraphs [0002] - [0143] * | 1-9 | TECHNICAL FIELDS SEARCHED (IPC)<br><br>A61B<br>G06N |

The present search report has been drawn up for all claims

| Place of search | Date of completion of the search | Examiner |
|---|---|---|
| The Hague | 13 February 2025 | Moran, Matthew |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 42 5045

This annex lists the patent family members relating to the patent documents cited in  the above-mentioned European search report.
The members are as contained in the European Patent Office EDP file on
The European Patent Office is in no way liable for these particulars which are merely given for the purpose of information.

13-02-2025

| Patent document cited in search report | Publication date | Patent family member(s) | | Publication date |
|---|---|---|---|---|
| US 2020104039 A1 | 02-04-2020 | CN | 112789578 A | 11-05-2021 |
| | | EP | 3857337 A1 | 04-08-2021 |
| | | KR | 20210058969 A | 24-05-2021 |
| | | US | 2020104039 A1 | 02-04-2020 |
| | | US | 2021255763 A1 | 19-08-2021 |
| | | US | 2023013680 A1 | 19-01-2023 |
| | | WO | 2020068455 A1 | 02-04-2020 |
| US 2023134166 A1 | 04-05-2023 | CN | 115943388 A | 07-04-2023 |
| | | US | 2021397929 A1 | 23-12-2021 |
| | | US | 2023134166 A1 | 04-05-2023 |
| | | WO | 2021257372 A1 | 23-12-2021 |

EPO FORM P0459

For more details about this annex : see Official Journal of the European Patent Office, No. 12/82